# EUROPEAN PATENT SPECIFICATION

(11) **EP 3 644 829 B1**
(45) Date of publication and mention of the grant of the patent: **28.07.2021**
(21) Application number: 18730737.6
(22) Date of filing: 08.06.2018
(51) Int. Cl.: A61B 3/107, A61B 3/10

(54) **OPHTHALMOLOGICAL APPARATUS**
OPHTHALMOLOGISCHES GERÄT
APPAREIL OPHTALMOLOGIQUE

(30) Priority: 29.06.2017 GB 201710461
(43) Date of publication of application: 06.05.2020
(73) Proprietor: Aston University, Birmingham West Midlands B4 7ET (GB)
(72) Inventor: WOLFFSOHN, James S., Birmingham West Midlands B4 7ET (GB); TULLOCH, Andrew, Birmingham West Midlands B4 7ET (GB); OBSZANSKI, Karl, Birmingham West Midlands B4 7ET (GB); DREW, Thomas, Birmingham West Midlands B4 7ET (GB)
(74) Representative: Mewburn Ellis LLP
(86) International application number: PCT/EP2018/065158
(87) International publication number: WO 2019/001928

(56) References cited:
- US-A1- 2015 131 055
- US-A1- 2016 000 322
- US-A1- 2017 042 421
- US-B1- 6 447 119

## Description

### FIELD OF THE INVENTION

The invention relates to an ophthalmological apparatus for inspecting a patient's eye. In particular, the invention relates to apparatus which involve projecting a light pattern onto the patient's eye.

### BACKGROUND TO THE INVENTION

In order to examine a patient's eye, an eye practitioner (e.g. ophthalmologist, optometrist or dispensing optician) will often use a slit lamp biomicroscope (also known as a slit lamp). The slit lamp enables the eye care practitioner to obtain a magnified view of various structures near the surface of the human eye, such as the eyelid, cornea, conjunctiva, sclera, iris and lens. The slit lamp is therefore a useful tool for diagnosing a wide variety of eye conditions, and it is currently one of the most widely used tools in the field of eye care.

A slit lamp works by producing a beam of light which can be adjustably moved and focused to image relevant portions of a patient's eye. The beam is produced by shining light from a light source through an aperture. The beam is then focused onto the patient's eye to illuminate a portion of the patient's eye. The aperture used is usually slit-shaped, such that the illuminated portion of the eye is slit-shaped. The eye is viewed through an optical line-up (e.g. similar to a microscope) that provides a magnified view of the illuminated portion of the eye. The slit lamp may also include a camera connected to record the magnified images of the patient's eye.

A conventional slit lamp instrument thus includes three main components: an illumination system, an observation system and a mechanical system which couples the illumination system to the observation system.

The illumination system includes a light source (e.g. a lamp) which produces light, a mechanical aperture (e.g. a slit) through which the light is shone in order to produce a light beam, filters (e.g. for amplifying light sensitive stains) and a series of optical components for focusing and positioning the light beam. The light beam can be projected onto a patient's eye to produce an image of the aperture on the patient's eye, thus illuminating a portion of the patient's eye. The portion of the eye which is illuminated, as well as the shape of the illuminated portion, can be adjusted by manually adjusting the shape and position of the mechanical aperture. For example, a moveable shutter on the mechanical aperture can be used to vary the size of the aperture. The light source emits optical radiation, usually in the visible spectrum, and may for example be a broadband light source. The colour of the light can be modified by placing a colour filter in front of the light beam.

The observation system includes a microscope having an objective lens and an eyepiece arranged to give an operator a magnified view of the patient's eye (typically up to 40x). The microscope is often a binocular microscope, in order to provide the operator with a stereoscopic view. The microscope can include multiple interchangeable objective lenses for switching between different levels of magnification. A camera port can be included so that a camera may be connected to the microscope in order to record images of the patient's eye for analysis.

The mechanical system couples the illumination system to the observation system. The mechanical system permits relative movement between the illumination and observation systems with respect to a base to which they are both connected. Typically, the mechanical system includes a three-axis positioning stage for accurately positioning the beam in relation to the patient's eye. The stage position is controlled manually using a set of knobs or a joystick. Additionally there may be some mechanism for adjusting the position of the observation system relative to the patient's eye, to ensure that the focal plane of the microscope can be aligned with the focal plane of the aperture image. The observation and illumination systems are coupled so that they are rotatable relative to the base about a common axis of rotation.

The mechanical system also includes a head rest to position the patient's face relative to the instrument. This will typically include a chin rest and a band against which the patient may place their forehead, both of which are vertically adjustable. Additionally, the mechanical system often includes a movable fixation target, on which an operator can ask the patient to focus during the examination procedure.

A conventional slit lamp is therefore a complex device including many moving parts which require significant manual adjustment and fine tuning in order to successfully carry out an examination of the patient's eye. For example, a slit lamp operator must adjust the head rest and fixation target in order to appropriately position the patient's eye relative to the instrument. The operator must then use the three-axis positioning stage to focus the light beam on the desired portion of the patient's eye. Finally, the microscope must be adjusted so that its focal plane coincides with the focal plane of the aperture image projected onto the patient's eye. If multiple portions of the patient's eye are to be examined, these adjustments may need to be repeated several times, which can lead to a time consuming examination procedure. If different filters or aperture sizes are to be used, this also requires a manual adjustment of the slit lamp.

Additionally, the curvature of the eye's surface causes a light pattern reflected by the eye's surface to be distorted. Different angles of incidence of the light beam relative to the eye's surface can lead to different levels of distortion. It can therefore be desirable to maintain a constant angle of incidence across a series of measurements, to ensure that the level of distortion remains constant. This facilitates data analysis procedures which involve the comparison of multiple measurements (e.g. using recorded images of the reflected patterns). For example, an operator of the slit lamp may wish to adjust the slit lamp such that the angle of incidence between the light beam and the eye's surface is as close as possible to 90 degrees. This can be difficult to achieve in practice however, as conventional slit lamps do not include a mechanism for measuring the angle of incidence relative to the curved cornea. The operator will therefore have to rely on his own judgement and ability to adjust the slit lamp in order to maintain a constant angle of incidence.

For these reasons, the operation of a conventional slit lamp is highly challenging, such that a skilled operator is required to carry out eye examinations with a slit lamp.

Prior art document US 6,447,119 discloses an ophthalmological apparatus for inspecting an eye and a method of viewing the surface of a subject's eye according to the preambles of claims 1 and 13 respectively. Prior art document US 2017/042421 discloses a modular unit for use as part of an ophthalmological apparatus according to the preamble of claim 11.

### SUMMARY OF THE INVENTION

At its most general, the present invention provides an ophthalmological apparatus with a digitally controllable mask for facilitating rapid and repeatable eye inspection. The digitally controllable mask may be a mask that is controlled by electronic signals to set and adjust the extent to which it blocks or transmits light from a light source. The apparatus may further comprise a light source and imaging device that are operable with the mask in a complementary fashion to aid efficient eye inspection.

According to a first aspect of the invention, there is provided an ophthalmological apparatus for inspecting an eye, the apparatus comprising: a light source; a mask arranged to selectively block transmission of light from the light source to form a mask pattern that is projectable onto a subject's eye, wherein the mask is adjustable based on a digital control signal to alter the mask pattern; and an imaging device for viewing the mask pattern on the subject's eye. The digital control signal may be provided by a suitable controller, e.g. computer, tablet, smartphone or other suitable processing apparatus, to enable the mask pattern projected onto the eye to be controlled in an automated or quasi-automated manner. Unlike conventional devices such as the slit lamp, the present invention does not require manual adjustment of moving parts to control the shape and position of the projected pattern, thus making the apparatus of the invention much simpler to operate. An advantage of the ophthalmological apparatus of the invention is that the observation procedure can be fully automated such that it requires minimal user intervention. Using the ophthalmological apparatus, images of the subject's eye can be recorded and analysed in order to determine various properties of the subject's eye.

The mask may selectively block transmission of light from the light source by having light-transmitting region(s) and regions which are opaque or cause reduced light transmission. For example, the mask pattern may be formed by a light-transmitting region in the shape of a slit, a ring, a series of concentric rings or any other complex two-dimensional pattern. The mask pattern may be time-varying. For example, in the case of a pattern including a light-transmitting slit, the slit can be swept from one side of the mask to another side of the mask. Additionally, the light transmission properties of the mask pattern may be wavelength-dependent, such that certain portions (or all) of the mask pattern may filter out particular wavelengths from the transmitted light, or tune the transmitted light to a particular wavelength. The mask may further be capable of displaying a fixation target for the patient to focus on during the measurement procedure. The fixation target may be movable, in order to cause the patient to move their eyes in a desired way during the measurement. In order to adjust the mask pattern the digital control signal is used. The position of the fixation target may also be based on the digital control signal. The digital control signal may be generated by a controller/processor which is integrated in the apparatus or which is in a separate device. Adjusting the mask pattern refers, for example, to varying parameters such as the shape, size, position, and/or colour of the light-transmitting region of the mask pattern. Therefore no three-axis stage or mechanical apertures are required to adjust the mask pattern, as is the case for a conventional slit lamp. This can greatly facilitate operation of the device.

The light source may be a conventional light source such as a projector lamp or a light panel. The light source is positioned such that light emitted from the light source can be transmitted through the mask and impinge on a subject which is on the opposite side of the mask to the light source. This enables the mask pattern to be projected onto the subject. By adjusting the mask pattern, the shape, size, position and/or colour of the mask pattern projected onto the subject's eye can be varied. The apparatus may include optical components before or after the mask to control the direction or beam shape of the emitted light. For example, the apparatus may include a collimator or similar, which can enable sharper images of the mask pattern to be obtained.

The imaging device serves to view the mask pattern on the subject's eye. The imaging device is arranged such that light reflected from the subject's eye forms an image at the imaging device which is viewable by a user. The imaging device can include any combination of conventional apparatus used for producing an image from incident light, such as an objective lens, a microscope, an image sensor or a camera. The imaging device may have an adjustable zoom and focus, for viewing features at different depths in the eye's surface and at different levels of magnification. The imaging device may also be configured to record images of the mask pattern on the subject's eye and store the images in a memory. The position of the imaging device may be adjusted with respect to the light source and mask in order to improve depth perception. More than one imaging device might be included, for example to enable 3-dimentional image reconstruction.

The apparatus may further include a face rest (e.g. chin rest and/or forehead rest) for positioning a patient's face relative to the apparatus. This ensures that the subject's face is held in place during the course of a measurement. The face rest may include a positioning stage (e.g. a three-axis stage) for setting the position of the patient's face relative to the apparatus at the beginning of a measurement.

The mask may comprise an array of independently controllable masking elements, each masking element being adjustable, e.g. based on the digital control signal, between a transmissive state for transmitting light from the light source and an opaque state for blocking transmission of light from the light source. The state of each masking element therefore determines the amount (i.e. intensity) of light from the light source which it can transmit. In certain embodiments, the state of the masking element may also determine the colour of the light transmitted. The mask pattern is adjusted by adjusting the state of one or more masking elements.

Each independently controllable masking element may be independently addressable, i.e. associated with a respective address (e.g. digital label) in order for it to be controlled individually via the digital control signal. The digital control signal may contain a series of masking element addresses and a series of state indicators, where each masking element address is associated with a respective state indicator. A state indicator indicates the state to which a masking element should be adjusted. Upon receipt of the digital control signal, the state of each masking element is adjusted in accordance with the state indicator which is associated with that masking element's address in the digital control signal. If the masking element addresses included in the digital control signal represent a subset of the masking elements in the mask, then only the states of the masking elements corresponding to the addresses included in the digital control signal are adjusted.

The array may be mounted in a frame for locating in an optical path from the light source to the subject's eye. The mask may comprise a liquid crystal display (LCD) window, i.e. an LCD screen mounted within a frame, where the masking elements correspond to the LCD screen's pixels. The light source may also be controllable. The light source comprises a light-emitting portion from which light is projected out of the apparatus, and wherein a relative position of the light-emitting portion and the mask is controllable to permit adjustment of an angle of incidence of the mask pattern projectable onto the subject's eye. In other words, when light from the light source is projected through the mask, it may form a light beam corresponding to the mask pattern. By moving the light-emitting portion of the light source relative to the mask, the direction of the beam can be varied, thus changing the angle at which the mask pattern impinges on the subject's eye. The light-emitting portion of the light source may refer to a region (e.g. a subregion) on the light source from which light can be emitted. The angle of incidence is defined as the angle between a beam incident on the subject's eye and the normal to the eye's surface at the point of incidence of the light beam on the eye's surface. By both adjusting the mask pattern and controlling the light-emitting portion of the light source, the position and angle of incidence of the projected mask pattern on the subject's eye can be controlled. This enables the mask pattern to be continuously swept across the subject's eye, and can allow for a wide and deep viewing field.

The light source may include an array of selectively activatable lighting elements. The relative position of the light-emitting portion and the mask may be controllable by selectively activating one or more of the lighting elements. For example, the array of selectively activatable lighting elements may be a panel array of LEDs or light bulbs. Activating one or more of the lighting elements may refer to causing the one or more lighting elements to emit light. The light-emitting portion can therefore be defined by the lighting elements which are activated. For example, a single lighting element may be activated, in which case the light-emitting portion of the light source can be defined as the position of the activated lighting element. In the case where multiple lighting elements are activated (e.g. a row of lighting elements), the light-emitting portion of the light source can be defined as an average position of the activated lighting elements. Different lighting elements can be activated sequentially, in order to move the light-emitting portion of the light source and adjust the angle of incidence of the mask pattern projected onto the subject's eye.

Alternatively or additionally, the light source may include a lighting element and a secondary mask arranged to selectively block transmission of light from the lighting element. The relative position of the light-emitting portion and the mask may be controllable by adjusting a position of a light-transmitting region of the secondary mask relative to the lighting element. The lighting element may serve to emit light, and may for example be a projector lamp or a light panel (such as an LED or OLED panel). The secondary mask may be arranged such that it is on a light path between the lighting element and the digitally controllable mask discussed above (referred to as the first mask below). Light from the light source which reaches the first mask will have passed through the secondary mask. In this configuration, the light-emitting portion of the light source can be defined by the position of the light-transmitting region (e.g. as a point in the light-transmitting region).

The secondary mask may also be digitally controllable, e.g. based on a second digital control signal. The secondary mask may have any of the features discussed herein with respect to the first mask. For example, the secondary mask may comprise an array of independently controllable masking elements analogous to those discussed above in relation to the first mask. The second digital control signal may indicate a shape, size and position of the light-transmitting region on the secondary mask.

The light source may be provided by lighting elements in the display of a tablet computer or smartphone. In such an embodiment, the tablet computer or smartphone may be removably attachable to the mask, e.g. via a supporting frame or the like. The tablet computer or smartphone may include software enabling it to produce light at a desired position on its screen and of a desired colour. The tablet computer or smartphone may be controlled remotely when it is installed in the ophthalmological apparatus (e.g. wirelessly or through a communication port), or a lighting sequence may be pre-programmed before it is installed in the apparatus. In one embodiment, the tablet computer or smartphone may be configured to control the mask, such that an entire measurement routine may be run by software on the tablet computer or smartphone, and any required image analysis may be performed by a processor therein. Using a tablet computer or smartphone as a light source in the ophthalmological apparatus provides a convenient and easily transportable solution, as no additional computer may be required to operate the apparatus.

The apparatus may comprise a polariser arranged to polarise light from the light source. The polariser may be provided in the mask or in the light source or in both the mask and the light source. The polariser may serve to reduce glare back into the imaging device or to allow investigation of the reflection of light from structures in the patient's eye. The polariser may also serve to increase colour saturation.

The imaging device may include: an image sensor for capturing a digital image, an optical line-up arranged to focus an image of the mask pattern that is formed on the subject's eye onto the image sensor, and a display for displaying the captured digital image. The optical line-up may comprise one or more optical elements (e.g. lenses or the like), e.g. an objective lens. The image sensor may for example be a CCD chip or other suitable image sensing device. The image sensor may be connected to the display such that images detected by the image sensor are shown on the display. The optical line-up may have an adjustable zoom and focus.

The image sensor may comprise a pair of independent image sensors disposed in a spaced relationship to enable stereoscopic images to be detected. This allows a three-dimensional image of the patient's eye to be reconstructed. In addition to allowing stereoscopic images to be detected, such a configuration may also enable the surfaces of both of a subject's eyes to be viewed simultaneously. The stereoscopic depth of the images can be enhanced by increasing the separation between the image sensors. For example, the stereoscopic depth of the images can be enhanced by separating the image sensors by more than the separation of human eyes, which is typically around 60 mm.

The imaging device may include a camera arranged to record an image of the mask pattern on the subject's eye. An objective lens may be used to focus light onto an image sensor of the camera. The imaging device may also include two cameras to allow stereoscopic images to be captured.

Where the imaging device is arranged to allow stereoscopic images to be captured, a stereoscopic image of the entire eye can be obtained by capturing images of different portions of the eye and stitching the images together. Using the stereoscopic image of the entire eye, a user may pan around and zoom into areas of interest to analyse a feature of the anterior eye (e.g. a scratch). Properties such as the depth and profile of the feature may be determined from the stereoscopic image.

The imaging device may be disposed behind the mask relative to the direction in which the mask pattern is emitted therethrough. The mask may include an aperture to permit the imaging device to view the mask pattern on the subject's eye. The aperture may be an actual hole in the mask, or it may be a light-transmitting region of the mask through which the imaging device can see. Where the aperture is a light-transmitting region of the mask, the size and position of the aperture may be varied, based on the digital control signal, in accordance with the requirements of a particular measurement. The aperture allows the imaging device to be located on an opposite side of the mask to the subject. In embodiments where the imaging device includes two image sensors or cameras, the mask may include two separate apertures so that each image sensor/camera can receive light reflected from the subject's eye.

The apparatus may comprise a pair of flat panels that are disposed substantially parallel to each other, whereby the apparatus takes the form of a cuboidal, which may be the size of a book. The mask may be contained within a first panel and the light source may be contained within a second panel. The two panels may have similar dimensions. The panels may be spaced by a distance which optimizes the range of angles through which the mask pattern can be swept across the subject's eye. In embodiments where the mask includes an aperture, the imaging device can be located between the light source and the mask, e.g. within the cuboidal body formed by the panels. Alternatively, the imaging device may be located behind the light source, such that the light source is located between the imaging device and the mask. In this case, an additional aperture in the light source may be necessary for the imaging device to see the subject's eye through.

The apparatus may include a controller configured to generate the digital control signal. The controller can for example generate the digital control signal based on a user input, or based on a pre-programmed measurement routine. The controller can further transmit the digital control signal to the mask, so that the mask may adjust the mask pattern based on the digital control signal. The controller may be integrated in the ophthalmological apparatus, or it may be included in an external control device. In the case where the controller is part of an external control device, the control device may be configured to communicate with the ophthalmological apparatus, for example wirelessly or via a cable. The controller and/or control device may include software which allows a user to set the mask pattern that is to be projected onto the subject's eye. The controller and/or control device may also enable the user to control the position of the fixation target. In some embodiments the controller/control device includes a touchscreen, such that the mask pattern may be adjusted via finger gestures on the touchscreen.

The controller may further be configured to generate the second digital control signal for controlling the light-emitting portion of the light source. The software may then enable the user to set the light-emitting portion of the light source and/or the colour of light emitted by the light source. Using the controller, a user may thus simultaneously control the light-emitting portion of the light source and the mask pattern, in order to project the mask pattern onto a relevant portion of the subject's eye and sweep the mask pattern across the subject's eye. The controller may also be configured to receive data (e.g. recorded images) from the imaging device and set one or more image capture parameters of the imaging device (e.g. zoom, focus, exposure time, filters). Recorded images can be analysed by the controller or a separate computing device to determine properties of the patient's eye such as corneal topography or tear film stability.

The controller/control device may include a memory module in which pre-programmed measurement routines are stored. The pre-programmed measurement routines may define sequences of mask patterns, light-emitting portions of the light source, and image capture parameters of the imaging device. The user can select a measurement routine, which causes the controller to generate a sequence of control signals for controlling the mask pattern, light source and imaging device according to the pre-programmed routine.

Furthermore, the controller may be configured to, in response to an analysis performed on a recorded image, control one or more of the mask, light source and imaging device to adjust a measurement parameter (e.g. by generating and transmitting corresponding control signals). The measurement parameters can include, but are not limited to: light-emitting portion of the light source; shape, size and position of the mask pattern; zoom, focus and exposure time of the imaging device.

The controller thus allows for centralised control of all the components of the ophthalmological apparatus, thus enabling automatic operation of the apparatus. This avoids much of the difficulty associated with operating conventional slit lamps.

According to a second aspect of the invention, there is provided a modular unit for use as part of an ophthalmological apparatus, the modular unit comprising: a frame for locating adjacent a subject's eye, the frame comprising: a mask mounted thereon; a first mounting portion arranged to receive a light source on the opposite side of the mask from the subject's eye; a second mounting portion arranged to receiving an imaging device, wherein the mask is arranged to selectively block transmission of light from the light source to form a mask pattern that is projectable onto a subject's eye, wherein the mask is adjustable based on a digital control signal to permit adjustment of the mask pattern, and wherein the second mounting portion is arranged to locate the imaging device in a position to view the mask pattern on the subject's eye.

The ophthalmological apparatus, of which the modular unit is used as a part, is based on the same principles as the ophthalmological apparatus discussed above. Accordingly, features of the first aspect of the invention above may be shared by the second aspect of the invention and are not discussed again.

An advantage of such a modular unit is that it can be used with a variety of light sources and imaging devices, such that the ophthalmological apparatus can be optimally configured to suit particular measurement requirements. Furthermore, this facilitates transport of the ophthalmological apparatus, as the components can be removed from the modular unit, thus making the modular unit compact and easily transportable.

The frame may be any suitable means for connecting the mask, light source and imaging device together. For example it may consist of an enclosure having slots in which the various components may be installed. The components may be removably attachable to the frame using suitable fastening means, such as, but not limited to, screws, clamps, clips and/or magnets. This enables components to be quickly and easily installed in, and removed from, the frame. The frame may also include a face rest for positioning the patient's face relative to the modular unit. The frame may be foldable.

In certain embodiments, the mask is removably attached to the frame. This further increases the flexibility and transportability of the modular unit. In further embodiments, a tablet computer or smartphone is removably attachable to the frame such that, when the tablet computer or smartphone is attached to the frame, lighting elements in the display of the tablet computer or smartphone provide the light source.

According to a third aspect of the invention, there is provided a method of viewing the surface of a subject's eye, the method including: positioning a mask between the subject's eye and a light source; transmitting a digital control signal to the mask to selectively block transmission of light from the light source and thereby project a mask pattern onto the subject's eye; and viewing, by an imaging device, the mask pattern on the subject's eye. This method may be partially or entirely carried out by a controller which is configured to control one or more of the mask, light source and imaging device, thus simplifying the procedure compared to that used with conventional slit lamps. The controller may be configured to generate the digital control signal and transmit it to the mask (e.g. wirelessly or via a cable). This enables an imaging procedure according to this method to be completely automated. This method may be used to operate the ophthalmological apparatus according to the first aspect of the invention. The features of the first aspect of the invention may therefore be shared with this aspect of the invention, and are not discussed again.

As mentioned above, the light source comprises a light-emitting portion from which light is projected. The method includes controlling a relative position of the light-emitting portion and the mask to select or adjust an angle of incidence of the mask pattern projected onto the subject's eye.

The method may include moving the mask pattern across the subject's eye, and controlling the angle of incidence of the mask pattern as it is moved over the subject's eye so that it is substantially normal to a surface of the eye.

The method may include recording, by the imaging device, an image of the mask pattern on the subject's eye; analysing the recorded image; and based on the analysis of the recorded image, controlling one or more of the mask, light source and imaging device to adjust a measurement parameter. For example the imaging device may record the image in a memory which is accessible by the controller. Measurement parameters can include, but are not limited to: a location of the light-emitting portion of the light source; shape, size and position of the mask pattern; zoom, focus and exposure time of the imaging device. This enables the measurement parameters to be automatically updated in real time, and so can allow for faster measurements. For example, if an analysis of a recorded image shows that the angle of incidence of the mask pattern projected onto the subject's eye is not the desired one, the light-emitting portion of the light source and the position of the mask pattern on the mask can be adjusted until the desired angle is obtained.

The method may include projecting a mask pattern of known dimensions onto the subject's eye, the subject's eye being located at a known distance from the imaging device; recording, using the imaging device, an image of the mask pattern on the subject's eye; and analysing the recorded image, in order to calculate the shape of the subject's eye. For example, the shape of the subject's eye can be calculated by comparing the distortions of the mask pattern formed on the patient's eye to the known dimensions of the projected mask pattern. This can provide a high resolution three-dimensional reconstruction of the surface of the patient's eye. The calculated shape of the subject's eye can be used, for example, when performing measurements such as tear film stability or corneal thickness/depth analysis, in order to improve the accuracy of the measurement.

The method may include controlling the mask and the light source in order to sweep the light pattern across the subject's eye. The calculated shape of the subject's eye may be used as an input parameter to control the sweep, such that an angle of incidence of the mask pattern on the patient's eye is kept constant throughout the sweep. The direction of the normal to the surface of the eye may vary by around 120 degrees across the surface of the eye. Compensating for the shape of the eye in the manner described allows features within the cornea to be observed more clearly, and their depth to be quantified more accurately.

The method may include recording, by the imaging device, a series of images of the mask pattern on the subject's eye; and analysing the series of recorded images in order to determine tear film stability and/or lipid layer thickness in the subject's eye. By analysing a series of images recorded at known time intervals, a user can study dynamics in the subject's eye in a non-invasive manner.

### BRIEF DESCRIPTION OF THE DRAWINGS

Examples of the invention are discussed below with reference to the accompanying drawings, in which:
Fig. 1 is a schematic diagram of a top view of an ophthalmological apparatus according the present invention set up to obtain an image of a patient's eye;
Fig. 2 is a schematic diagram showing a front view of a mask displaying a pattern for selectively transmitting light and a fixation target;
Fig. 3 is a schematic diagram showing a top view of an ophthalmological apparatus according to the present invention, where the sweeping of a projected light beam is illustrated; and
Fig. 4 is a schematic diagram showing a side view of an ophthalmological apparatus according to the present invention.

### DETAILED DESCRIPTION

The invention avoids the complexity of conventional slit lamps by providing an apparatus whose operation can be entirely automated. Thus, the ophthalmological apparatus of the invention does not require a skilled user in order to examine a patient's eye, making eye examinations more accessible and easier to carry out. The concept behind the ophthalmological apparatus of the invention is to project a mask pattern onto a patient's eye, where the mask pattern can be adjusted using a digital control signal. The ophthalmological apparatus of the invention therefore simplifies the initial positioning step, and for example does not require multiple moving parts such as a three-axis stage or mechanical aperture in order to control the position and shape of the pattern projected onto the patient's eye.

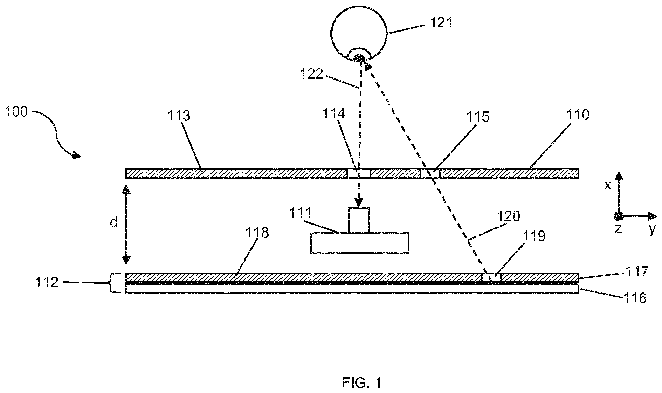

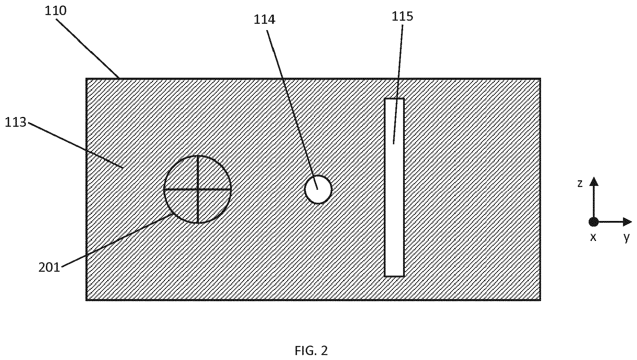

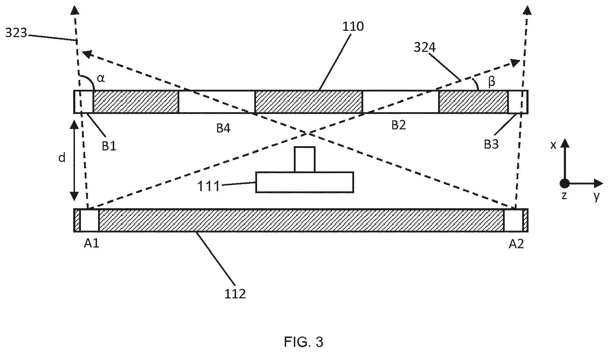

Fig. 1 shows a schematic diagram of an ophthalmological apparatus 100 according to the invention which is set up to image the surface and anterior of a patient's eye 121. The ophthalmological apparatus 100 includes three main components: a primary mask 110, an imaging device 111 and a light source 112. In this embodiment the primary mask 110 and the light source 112 are formed by flat rectangular panels, and are disposed such that they are substantially parallel and spaced by a distance d. In other embodiments, one or both of the primary mask 110 and the light source 112 may be curved panels. As the surface of the eye is curved, curved panels may give superior viewing and projection capabilities. The imaging device 111 is positioned between the primary mask 110 and the light source 112. The view shown in Fig. 1 is a top view of the device, such that the primary mask 110 and the light source 112 have a height along the z-direction (see Figs. 2 and 3). Each of the figures uses the same coordinate system, shown by the axes included in each figure.

The primary mask 110 is a screen with selectively adjustable transparency, such as a transparent LCD screen or other similar technology. The primary mask 110 includes a two-dimensional array of independently controllable masking elements, each one having an independently controllable opacity level. The primary mask 110 is capable of displaying complex patterns having regions which are light-transmitting and regions which are opaque or cause reduced light transmission. Patterns are produced by individually controlling the opacity level of each masking element. The masking elements may be made from any structure capable of exhibiting variable opacity, e.g. an LCD pixel, a polariser, a smart glass element, etc.

In the example shown in Fig. 1, the primary mask 110 includes an opaque region 113, shown as the striped section. The pattern also includes two light-transmitting regions: an aperture 114 positioned in front of the imaging device 111 for the imaging device to look through, and a slit shape 115 for transmitting light from the light source 112. This is a two-dimensional pattern, as illustrated by the front view of the primary mask shown in Fig. 2. The pattern displayed by the primary mask 110 can easily be changed by adjusting the opacity levels of the masking elements, such that there is no need to move the mask itself relative to the patient's eye during a measurement. Additionally, screens with selectively adjustable transparency such as transparent LCD screens can have very fast response times (e.g. less than 10 ms), such that the displayed pattern may be adjusted and varied to high accuracy in real time during a measurement. For example, the slit 115 shown in Figs 1 and 2 could be swept back and forth across the screen along the y-direction, or the size and/or position of the aperture 114 could be varied. Other more complex patterns can also be produced, such as placido rings which can be used to measure tear film stability or the shape of the cornea. Additionally, the primary mask 110 is also capable of displaying a fixation target 201, as illustrated in Fig. 2. This fixation target is movable, and serves as a point for the patient to focus on during the examination procedure.

The pattern produced by the primary mask 110 may be based on a control signal sent to the mask 110, either wirelessly or via a communication port. For example, a computing device (not shown) can be connected to the primary mask 110, where the computing device includes software for controlling the pattern displayed by the primary mask 110. Alternatively, the primary mask 110 may itself include a controller configured to control the pattern it produces. The user may determine the pattern to be produced by the primary mask 110 by making inputs on the computing device and/or controller. In cases where the computing device and/or controller includes a touchscreen, inputs can include finger gestures on the touchscreen. This can provide a user-friendly interface for controlling the ophthalmological apparatus.

Turning back to Fig. 1, the light source 112 includes a light panel 116 and a secondary mask 117. The light panel 116 can be any commercially available light panel, such as an LED light panel. The secondary mask 117 is a screen with selectively adjustable transparency, and works in the same way as the one used for the primary mask 110 described above (i.e. it has an array of independently controllable masking elements). For example the secondary mask 117 may be a transparent LCD screen or other similar technology. The light panel 116 is coupled to secondary mask 117 such that light emitted from the light panel 116 passes through the secondary screen 117 before it impinges on the primary mask 110. The light source 112 may for example be a backlit LCD screen, OLED screen, LED array, diffuse light panel or some similar technology. In the example shown in Fig. 1, the secondary mask 117 includes an opaque region 118 and a light-transmitting region 119. The light-transmitting region of the secondary mask 117 defines a light-emitting portion of the light source 112, as light will only be emitted from the light source 112 at the light-transmitting region 119 of the secondary mask 117. The light-emitting portion of the light source 112 can be varied by modifying the position and/or shape of the light-transmitting region 119 of the secondary mask 117. This is done by adjusting the opacity level of the secondary mask's masking elements. There can be multiple light-emitting portions, depending on the requirements of the measurement. The brightness of the light source can be adjusted by varying the opacity of the light-transmitting region. Note that other types of lamp may be used instead of the light panel 116 shown in Fig. 1. For example, a projector lamp could be shined through the secondary mask 117.

Fig. 1 illustrates how an image of the surface of a patient's eye 121 may be obtained using the ophthalmological apparatus 100. A light ray 120 emitted by the light panel 116 is transmitted through the light-transmitting region 119 of the secondary mask 117 and then transmitted through the light-transmitting slit 115 in the pattern displayed by the primary mask 110. The light ray 120 then impinges on the surface of the patient's eye 121. A reflected light ray 122 passes through the light-transmitting aperture 114 in the primary mask 110 to produce an image at the imaging device 111. Using ophthalmological apparatus 100, the user can therefore project an image of the pattern displayed by primary mask 110 onto the patient's eye 121, and view an image of the pattern reflected by the patient's eye 121 with the imaging device 111. This enables the user to observe in detail the different structures of the surface of the patient's eye, such as the cornea, eyelid, sclera, conjunctiva and iris.

In this example, the imaging device 111 is a camera having adjustable zoom and focus. The camera can be any conventional camera such as a digital camera. The camera is configured to record images of the patient's eye 121 through the aperture 114 in the primary mask 110. By adjusting the focus of the camera, images of structures at different depths in the eye's surface can be obtained. The zoom enables the user to view the eye's surface at different levels of magnification. The camera focus and zoom can be controlled automatically, for example by software on the computing device. Typically magnification levels between 6x and 40x may be obtained, although higher magnification may also be obtained.

Optionally, the imaging device 111 can include a second camera such that stereoscopic images of the patient's eye can be captured. In such a configuration, the second camera is placed at the same x-coordinate as the first camera, and displaced relative to the first camera by a known distance along the y-direction, according to the coordinate system shown in Fig. 1. Depending on the configuration of the two cameras, it may be necessary to include a second aperture in the primary mask 110 for the second camera to see through. Depth perception may be improved by increasing the separation between the two cameras. For example, the cameras may be separated by 65 mm or more. Alternatively, a microscope could be used in place of the camera, in order to obtain high levels of magnification.

In certain embodiments, it may be advantageous to position the imaging device 111 behind the light source 112, such that the light source 112 is positioned between the imaging device 111 and the primary mask 110. In such a case a further aperture may be provided in the light source 112 for the imaging device 111 to see through. Alternatively, the imaging device could be placed in another location, for example to the side of the primary mask 110 and light source 112, such that no apertures are necessary.

Fig. 3 illustrates how, by sweeping both the light-transmitting region on the primary mask 110 and the light-emitting portion of the light source 112, a wide and deep viewing field can be obtained and the angle of incidence on the patient's eye can be controlled. The angle of incidence is defined as the angle between the incident beam and the normal to the eye's surface at the point of incidence of the light beam on the eye's surface. The dashed lines show possible light trajectories for light emitted by the light source 112. Depending on the relative positions of the light-emitting portion and the light-transmitting region of the primary mask 110, the light may traverse the primary mask 110 a different angles. For example as shown in Fig. 3, if the light source's 112 light-emitting portion is at position A1 and the light-transmitting region of the primary mask 110 is at position B1, the angle α between the transmitted light ray 323 and the primary mask 110 will be close to 90 degrees. However, if the light-emitting portion is kept at position A1 and the light-transmitting region of the primary mask 110 is moved to position B2, the angle β between the transmitted light ray 324 and the primary mask 110 is much less than 90 degrees. Similar results can be obtained by setting the light-emitting portion to position A2, and moving the position of the light-transmitting region of the primary mask 110 between positions B3 and B4. Note that the angle of incidence on the patient's eye is related to the angle between the transmitted light and the primary mask 110, and it can be calculated knowing the position of the eye and its shape.

The person skilled in the art will appreciate that the angle between the transmitted light and the primary mask 110 can be varied continuously between an upper limit and a lower limit, by controlling the position of the light-transmitting region on the primary mask 110 and the light-emitting portion of the light source 112. The upper and lower limit will depend on the dimensions of the light source 112 and the primary mask 110, as well as the position of the imaging device 111. This enables the user to adjust the angle of incidence of the light pattern projected onto the patient's eye 121 in real time during a measurement. The primary mask 110 and the light source 112 can both be controlled by the same computing device, such that the angle of incidence of the transmitted light can be automatically controlled. By analysing recorded images of the pattern reflected from the patient's eye 121 (e.g. placido rings or a swept slit), software on the computing device can determine the angle of incidence and, if necessary, adjust the light-emitting portion and the pattern produced by the primary mask 110 in order to set the angle of incidence to a predetermined angle (for example set by the user in the software). Additionally, if the image analysis shows that the patient's eye is not correctly oriented, the software can cause the fixation target on the primary mask 110 to move until the correct orientation is achieved. The orientation between imaging device and ocular surface for different angles of gaze can also be kept constant by moving the fixation target.

In some cases, lighting elements in a display of a tablet computer or smartphone can be used as the light source 112. In such a scenario, the tablet computer may replace the light panel 116 and secondary mask 117 shown in Fig. 1. The tablet or smartphone can have software installed thereon enabling it to produce light at a desired position on its screen (i.e. to control the light-emitting portion) and of a desired colour. The tablet computer or smartphone can be controlled remotely when it is installed in the ophthalmological apparatus (e.g. wirelessly or through a communication port), or a lighting sequence may be pre-programmed before it is installed in the apparatus. Advantageously, the tablet computer or smartphone itself can be used as the computing device which controls the measurement and performs the image analysis, by configuring the tablet computer or smartphone to control the imaging device 111 and the primary mask 110.

One or both of the primary mask 110 and the secondary mask 117 may be capable of acting as a filter to filter out particular wavelengths from the transmitted light, or tune the transmitted light to a particular wavelength. This is achieved by making the light transmission properties of the light-transmitting region dependent on the wavelength of the transmitted light. For example, if the secondary mask 117 is a transparent LCD screen having red, green and blue pixels, the light-transmitting region 119 of the secondary mask 117 can be set to act as a red-free filter by switching all of the screen's red pixels off. This can be used to increase the contrast in pictures taken of the eye's surface, as blood vessels will appear as black. The wavelength of the transmitted light can also be tuned to a peak wavelength for viewing an ophthalmic dye (for example fluorescein) which has been instilled into the patient's eye.

Filtering may also be applied when processing images from the imaging device 111. This may be the done where the imaging device 111 is an image sensor/camera having multiple sets of pixels, each set of pixels being sensitive to a particular range of wavelengths (e.g. the image sensor may have red-, green- and blue-sensitive pixels). Then, an image taken by a subset of the pixels (e.g. the red-sensitive pixels) may be extracted to effectively filter out light from the other wavelength ranges.

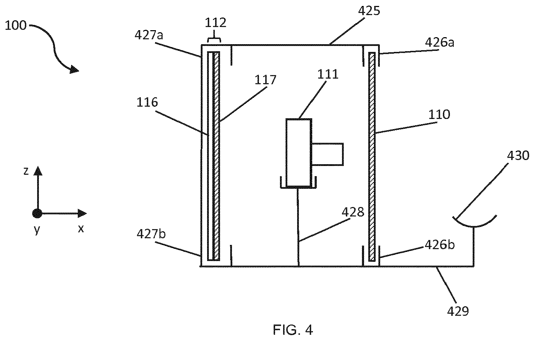

A side view of the ophthalmological apparatus 100 is shown in Fig. 4. The coordinate system in Fig. 4 corresponds to those shown in the previous figures. As in Figs. 1-3, the imaging device 111 is positioned between the primary mask 110 and the light source 112. Additionally, a frame 425 is shown for holding the device's components in their relative positions. The frame 425 includes slots 426a, 426b and 427a, 427b for holding the primary mask 110 and the light source 112 respectively, as well as a stand 428 for holding the imaging device 111. An arm 429 extends in front of the primary mask 110 for positioning a face rest 430 configured to position the patient's face and hold it in place during a measurement. The face rest 430 may include a chin rest on which the patient may place their chin, a head rest (not shown) against which the patient may place their forehead and/or other components suitable for holding and positioning the patient's face relative to the ophthalmological apparatus 100. The frame 425 may form an enclosure such that only the front face of the primary mask 110 is exposed, in order to prevent light from leaking in during the measurement. The frame 425 is shown for illustrative purposes only, and many other alternative frame configurations are possible.

Some or all of the components of the ophthalmological apparatus may be removably attachable to the frame, i.e. they can easily be installed in, and removed from, the frame, for example using fastening means such as screws, clamps and/or clips. This can be particularly advantageous, as it allows a tablet computer or smartphone to easily be installed and used as the lighting source 112, as described above. This makes the device highly versatile and user friendly.

According to certain embodiments of the invention, there is provided a modular unit composed of the primary mask 110 and a frame, wherein an imaging device 111 and a light source 112 are removably attachable to the frame to form an ophthalmological apparatus as described above in relation to Figs. 1-4. The frame can be similar to frame 425 shown in Fig. 4, and enables quick and easy installation and removal of the different components (for example using fastening means such as screws, clamps and/or clips). The modular unit can therefore be used with a variety of different light sources and imaging devices, such that the ophthalmological apparatus can be optimally configured to suit particular measurement requirements. Additionally this facilitates transport of the ophthalmological apparatus, as the different components can be removed from the modular unit, making the modular unit compact and easily transportable.

The ophthalmological apparatus 100 of the present invention can be used to perform many different types of measurement on the eye, in addition to producing images of the eye's surface as described above. For example, the ophthalmological apparatus 100 can be used to produce a three-dimensional reconstruction of the patient's eye 121. This is done by projecting a pattern of known dimensions (such as placido rings) onto the patient's eye 121, which is at a known distance from the imaging device 111, and recording an image of the pattern formed on the patient's eye 121 with the imaging device 111. An algorithm then analyses the reflected pattern and, based on the distortions in the reflected pattern, calculates the shape of the eye's surface (for example by performing a fit of the ring width/separation in multiple meridians). Once the shape of the eye's surface has been determined, this can be used as an input in the software which controls the primary mask 110 and the light source 112. This enables the software to calculate the angle of incidence of a pattern projected onto the eye, such that the angle of incidence can be set to a desired angle by adjusting the primary mask 110 and the light source 112. In this manner, for example, a pattern can be swept across the eye's surface whilst keeping the angle of incidence of the light relative to the eye's surface constant. The calculation of the shape of a patient's eye can be automatically performed at the beginning of an examination procedure, to improve the accuracy of subsequent measurements on the patient's eye 121.

The ophthalmological apparatus 100 can also be used to study dynamics in the patient's eye 121, by recording a sequence of images at set time intervals. For example, tear film stability and lipid layer thickness and dynamics can be measured in a non-invasive manner, by projecting a pattern of known dimensions (such as placido rings) onto the patient's eye 121 and analysing changes in the reflected pattern and/or interference patterns over time.

The examples shown in the Figures are for illustration purposes only, and the relative dimensions of the various components are not accurately portrayed. The invention is defined in the appended claims.

## Claims

1. An ophthalmological apparatus (100) for inspecting an eye, the apparatus comprising:
a light source (112);
a mask (110) arranged to selectively block transmission of light from the light source (112) to form a mask pattern that is projectable onto a subject's eye (121), wherein the mask (110) is adjustable based on a digital control signal to alter the mask pattern; and
an imaging device (111) for viewing the mask pattern on the subject's eye,
**characterised in that** the light source (112) comprises a light-emitting portion from which light is projected out of the apparatus, and wherein a relative position of the light-emitting portion and the mask (110) is controllable to permit adjustment of an angle of incidence of the mask pattern projectable onto the subject's eye (121).

2. An ophthalmological apparatus (100) according to claim 1, wherein the mask (110) comprises an array of independently controllable masking elements, each masking element being adjustable, based on the digital control signal, between a transmissive state for transmitting light from the light source (112) and an opaque state for blocking transmission of light from the light source (112), and optionally:
wherein the array is mounted in a frame (425) for locating in an optical path from the light source (112) to the subject's eye (121), and/or
wherein each independently controllable masking element is independently addressable.

3. An ophthalmological apparatus (100) according to claim 1 or claim 2, wherein the mask comprises a liquid crystal display window.

4. An ophthalmological apparatus (100) according to claim 1, wherein the light source (112) includes an array of selectively activatable lighting elements, and wherein the relative position of the light-emitting portion and the mask (110) is controllable by selectively activating one or more of the lighting elements; or
wherein the light source (112) includes a lighting element (116) and a secondary mask (117) arranged to selectively block transmission of light from the lighting element (116), and wherein the relative position of the light-emitting portion and the mask (110) is controllable by adjusting a position of a light-transmitting region of the secondary mask (117) relative to the lighting element, and optionally wherein the secondary mask (117) is controllable based on a second digital control signal.

5. An ophthalmological apparatus (100) according to any preceding claim, wherein the light source (112) is provided by lighting elements in the display of a tablet computer or smartphone.

6. An ophthalmological apparatus (100) according to any preceding claim comprising a polariser arranged to polarise light from the light source (112), and
optionally the polariser is provided in the mask (110) and/or the light source (112).

7. An ophthalmological apparatus (100) according to any preceding claim, wherein the imaging device (111) includes:
an image sensor for capturing an digital image,
an optical line-up arranged to focus an image of the mask pattern that is formed on the subject's eye onto the image sensor, and
a display for displaying the captured digital image, and
optionally the image sensor comprises a pair of independent image sensors disposed in a spaced relationship to enable stereoscopic images to be detected.

8. An ophthalmological apparatus (100) according to any preceding claim, wherein the imaging device (111) includes a camera arranged to record an image of the mask pattern on the subject's eye, and/or
wherein the imaging device (111) is disposed behind the mask (110) relative to the direction in which the mask pattern is emitted therethrough, and wherein the mask includes an aperture to permit the imaging device to view the mask pattern on the subject's eye (121).

9. An ophthalmological apparatus (100) according to any preceding claim, wherein the mask (110) is contained within a first panel and the light source (112) is contained within a second panel, the two panels being arranged such that they are substantially parallel.

10. An ophthalmological apparatus (100) according to any preceding claim further comprising a controller configured to generate the digital control signal,
wherein, optionally, the controller includes a processor and memory storing software instructions, wherein, when executed by the processor, the software instructions are arranged to control operation of the apparatus and/or the controller is operably connected to the imaging device (111) to control capture of an image of the mask pattern.

11. A modular unit for use as part of an ophthalmological apparatus (100), the modular unit comprising:
a frame (425) for locating adjacent a subject's eye (121), the frame (425) comprising:
a mask (110) mounted thereon;
a first mounting portion (427a, 427b) arranged to receive a light source (112), the light source (112) comprising a light-emitting portion from which light is projected, on the opposite side of the mask (100) from the subject's eye (121);
a second mounting portion (428) arranged to receiving an imaging device (111), wherein the second mounting portion (428) is arranged to locate the imaging device (111) in a position to view the mask pattern on the subject's eye (121),
wherein, when the light source is mounted in the first mounting portion, the mask (110) is arranged to selectively block transmission of light from the light source (112) to form a mask pattern that is projectable onto a subject's eye (121), **characterised in that** the mask (110) is adjustable based on a digital control signal to permit adjustment of the mask pattern and wherein a relative position of the light-emitting portion and the mask is controllable to permit adjustment of an angle of incidence of the mask pattern projectable onto the subject's eye.

12. A modular unit according to claim 11, wherein the mask (110) is removably attached to the frame, and/or
wherein the second mounting portion (428) is arranged to receive a tablet computer or smartphone, wherein the tablet computer or smartphone provides the light source and/or imaging device.

13. A method of viewing the surface of a subject's eye (121), the method including:
positioning a mask (110) between the subject's eye (121) and a light source (112);
transmitting a digital control signal to the mask (110) to selectively block transmission of light from the light source and thereby project a mask pattern onto the subject's eye (121); and
viewing, by an imaging device (111), the mask pattern on the subject's eye (121),
**characterised in that** the light source (112) comprises a light-emitting portion from which light is projected, and wherein the method includes controlling a relative position of the light-emitting portion and the mask (110) to select or adjust an angle of incidence of the mask pattern projected onto the subject's eye (121) .

14. A method according to claim 13 including:
moving the mask pattern across the subject's eye (112), and
controlling the angle of incidence of the mask pattern as it is moved over the subject's eye so that it is substantially normal to a surface of the eye; and/or
recording, by the imaging device, an image of the mask pattern on the subject's eye (121);
analysing the recorded image; and
based on the analysis of the recorded image, controlling one or more of the mask (110), light source (112) and imaging device (111) to adjust a measurement parameter.

15. A method according to claim 13 or claim 14, further including:
projecting a mask pattern of known dimensions onto the subject's eye (121), the subject's eye (121) being located at a known distance from the imaging device (111);
recording, using the imaging device (111), an image of the mask pattern on the subject's eye (121); and
analysing the recorded image, in order to calculate the shape of the subject's eye (121), and optionally further including:
controlling the mask (110) and the light source (112) in order to sweep the light pattern across the subject's eye (121),
wherein the calculated shape of the subject's eye (121) is used as an input parameter to control the sweep, such that an angle of incidence of the mask pattern on the patient's eye (121) is kept constant throughout the sweep; and/or
recording, by the imaging device (111), a series of images of the mask pattern on the subject's eye (121), and
analysing the series of recorded images in order to determine tear film stability and/or lipid layer thickness in the subject's eye (121).

## Patentansprüche

1. Ophthalmologische Vorrichtung (100) zum Untersuchen eines Auges, wobei die Vorrichtung Folgendes umfasst:
eine Lichtquelle (112);
eine Maske (110), die ausgebildet ist, um die Transmission von Licht von der Lichtquelle (112) selektiv zu blockieren, um ein Maskenmuster zu bilden, das auf das Auge (121) eines Individuums projizierbar ist, wobei die Maske (110) basierend auf einem digitalen Steuersignal einstellbar ist, um das Maskenmuster zu ändern; und
eine Bildgebungsvorrichtung (111) zum Betrachten des Maskenmusters auf dem Auge des Individuums,
**dadurch gekennzeichnet, dass**
die Lichtquelle (112) einen lichtemittierenden Abschnitt umfasst, von dem aus Licht aus der Vorrichtung hinaus projiziert wird, und wobei eine relative Position des lichtemittierenden Abschnitts und der Maske (110) steuerbar ist, um das Einstellen eines Einfallswinkels des Maskenmusters, das auf das Auge (121) des Individuums projizierbar ist, zu erlauben.

2. Ophthalmologische Vorrichtung (100) nach Anspruch 1, wobei die Maske (110) eine Anordnung von unabhängig steuerbaren maskierenden Elementen umfasst, wobei jedes maskierende Element basierend auf dem digitalen Steuersignal zwischen einem Transmissionszustand für die Transmission von Licht von der Lichtquelle (112) und einem nichtdurchlässigen Zustand für das Blockieren der Transmission von Licht von der Lichtquelle (112) einstellbar ist und gegebenenfalls:
wobei die Anordnung zum Anordnen in einem optischen Pfad von der Lichtquelle (112) zu dem Auge (121) des Individuums in einem Rahmen (425) angebracht ist, und/oder
wobei jedes unabhängig steuerbare maskierende Element unabhängig adressierbar ist.

3. Ophthalmologische Vorrichtung (100) nach Anspruch 1 oder Anspruch 2, wobei die Maske ein Flüssigkristall-Anzeigefenster umfasst.

4. Ophthalmologische Vorrichtung (100) nach Anspruch 1, wobei die Lichtquelle (112) eine Anordnung selektiv aktivierbarer Beleuchtungselemente umfasst und wobei die relative Position des lichtemittierenden Abschnitts und der Maske (110) durch selektives Aktivieren eines oder mehrerer der Beleuchtungselemente steuerbar ist; oder
wobei die Lichtquelle (112) ein Beleuchtungselement (116) und eine sekundäre Maske (117) umfasst, die ausgebildet ist, um die Transmission von Licht von dem Beleuchtungselement (116) selektiv zu blockieren und wobei die relative Position des lichtemittierenden Abschnitts und der Maske (110) durch Einstellen einer Position eines lichttransmittierenden Bereichs der sekundären Maske (117) relativ zu dem Beleuchtungselement steuerbar ist und wobei die sekundäre Maske (117) gegebenenfalls basierend auf einem zweiten digitalen Steuersignal steuerbar ist.

5. Ophthalmologische Vorrichtung (100) nach einem der vorangegangenen Ansprüche, wobei die Lichtquelle (112) durch Beleuchtungselemente in der Anzeige eines Tabletcomputers oder einem Smartphone bereitgestellt ist.

6. Ophthalmologische Vorrichtung (100) nach einem der vorangegangenen Ansprüche, die einen Polarisator umfasst, der ausgebildet ist, um Licht von der Lichtquelle (112) zu polarisieren und
der Polarisator gegebenenfalls in der Maske (110) und/oder der Lichtquelle (112) bereitgestellt ist.

7. Ophthalmologische Vorrichtung (100) nach einem der vorangegangenen Ansprüche, wobei die Bildgebungsvorrichtung (111) Folgendes umfasst:
einen Bildsensor zum Aufnehmen eines digitalen Bilds,
eine optische Anordnung, die ausgebildet ist, um ein Bild des Maskenmusters, das auf dem Auge des Individuums auf dem Bildsensor ausgebildet ist, zu fokussieren und
eine Anzeige zum Anzeigen des aufgenommenen digitalen Bildes und
gegebenenfalls der Bildsensor ein Paar von unabhängigen Bildsensoren umfasst, die in einer beabstandeten Beziehung angeordnet sind, damit sie stereoskopische Bilder detektieren können.

8. Ophthalmologische Vorrichtung (100) nach einem der vorangegangenen Ansprüche, wobei die Bildgebungsvorrichtung (111) eine Kamera umfasst, die ausgebildet ist, um ein Bild des Maskenmusters auf dem Auge des Individuums aufzunehmen, und/oder
wobei die Bildgebungsvorrichtung (111) hinter der Maske (110) relativ zu der Richtung, in der das Maskenmuster durch diese hindurch emittiert wird, angeordnet ist und wobei die Maske eine Öffnung umfasst, um der Bildgebungsvorrichtung zu ermöglichen, das Maskenmuster auf dem Auge (121) des Individuums zu betrachten.

9. Ophthalmologische Vorrichtung (100) nach einem der vorangegangenen Ansprüche, wobei die Maske (110) innerhalb einer ersten Platte enthalten ist und die Lichtquelle (112) innerhalb einer zweiten Platte angeordnet ist, wobei die zwei Platten so ausgebildet sind, dass sie im Wesentlichen parallel sind.

10. Ophthalmologische Vorrichtung (100) nach einem der vorangegangenen Ansprüche, die ferner eine Steuerung umfasst, die ausgelegt ist, um ein digitales Steuersignal zu erzeugen,
wobei die Steuerung gegebenenfalls einen Prozessor und Speichersoftwareanweisungen umfasst, wobei die Softwareanweisungen, wenn sie durch den Prozessor ausgeführt werden, so ausgebildet sind, dass sie den Betrieb der Vorrichtung steuern, und/oder die Steuerung mit der Bildgebungsvorrichtung (111) wirkverbunden ist, um das Aufnehmen eines Bilds des Maskenmusterns zu steuern.

11. Modulare Einheit zur Verwendung als Teil einer ophthalmologischen Vorrichtung (100), wobei die modulare Einheit Folgendes umfasst:
einen Rahmen (425) zum Anordnen benachbart zu einem Auge (121) eines Individuums, wobei der Rahmen (425) Folgendes umfasst:
eine Maske (110), die auf diesem angebracht ist;
einen ersten Anbringungsabschnitt (427a, 427b), der ausgebildet ist, um eine Lichtquelle (112) aufzunehmen, wobei die Lichtquelle (112) einen lichtemittierenden Abschnitt umfasst, von dem aus auf der entgegengesetzten Seite der Maske (100) von dem Auge (121) des Individuums Licht projiziert wird,
einen zweiten Anbringungsabschnitt (428), der ausgebildet ist, um eine Bildgebungsvorrichtung (111) aufzunehmen, wobei der zweite Anbringungsabschnitt (428) ausgebildet ist, um die Bildgebungsvorrichtung (111) in einer Position, in der das Maskenmuster auf dem Auge (121) des Individuums betrachtet werden kann, anzuordnen,
wobei, wenn die Lichtquelle in dem ersten Anbringungsabschnitt angebracht ist, die Maske (110) ausgebildet ist, um die Transmission von Licht von der Lichtquelle (112) selektiv zu blockieren, um ein Maskenmuster zu bilden, das auf das Auge (121) eines Individuums projizierbar ist,
**dadurch gekennzeichnet, dass**
die Maske (110) basierend auf einem digitalen Steuersignal einstellbar ist, um das Einstellen des Maskenmusters zu erlauben und wobei eine relative Position des lichtemittierenden Abschnitts und der Maske steuerbar ist, um das Einstellen eines Einfallswinkels des Maskenmusters, das auf das Auge des Individuums projizierbar ist, zu erlauben.

12. Modulare Einheit nach Anspruch 11, wobei die Maske (110) entfernbar mit dem Rahmen verbunden ist und/oder
wobei der zweite Anbringungsabschnitt (428) so angeordnet ist, dass er einen Tabletcomputer oder ein Smartphone aufnehmen kann, wobei der Tabletcomputer oder das Smartphone die Lichtquelle und/oder die Bildgebungsvorrichtung bereitstellt.

13. Verfahren zum Betrachten der Oberfläche des Auges (121) eines Individuums, wobei das Verfahren Folgendes umfasst:
das Positionieren einer Maske (110) zwischen dem Auge (121) des Individuums und einer Lichtquelle (112);
das Übertragen eines digitalen Steuersignals an die Maske (110), um die Transmission von Licht von der Lichtquelle selektiv zu blockieren und dadurch ein Maskenmuster auf das Auge (121) des Individuums zu projizieren; und
das Betrachten des Maskenmusters auf dem Auge (121) des Individuums durch eine Bildgebungsvorrichtung (111),
**dadurch gekennzeichnet, dass**
die Lichtquelle (112) einen lichtemittierenden Abschnitt aufweist, von dem aus Licht projiziert wird, und wobei das Verfahren das Steuern einer relativen Position des lichtemittierenden Abschnitts und der Maske (110) umfasst, um einen Einfallswinkel des auf das Auge (121) des Individuums projizierten Maskenmusters auszuwählen oder einzustellen.

14. Verfahren nach Anspruch 13, das Folgendes umfasst:
das Bewegen des Maskenmusters über das Auge (112) des Individuums, und
das Steuern des Einfallswinkels des Maskenmusters, während es über das Auge des Individuums bewegt wird, so dass es im Wesentlichen zu einer Oberfläche des Auges normal steht; und/oder
das Aufnehmen eines Bildes des Maskenmusters auf dem Auge (121) des Individuums durch die Bildgebungsvorrichtung;
das Analysieren des aufgenommenen Bildes; und,
basierend auf der Analyse des aufgenommenen Bildes, das Steuern eines oder mehrerer der Maske (110), der Lichtquelle (112) und der Bildgebungsvorrichtung (111), um einen Messparameter einzustellen.

15. Verfahren nach Anspruch 13 oder Anspruch 14, das ferner Folgendes umfasst:
das Projizieren eines Maskenmusters mit bekannten Dimensionen auf das Auge (121) des Individuums, wobei sich das Auge (121) des Individuums in einem bekannten Abstand von der Bildgebungsvorrichtung (111) entfernt befindet;
das Aufnehmen eines Bildes des Maskenmusters auf dem Auge (121) des Individuums unter Verwendung der Bildgebungsvorrichtung (111) und
das Analysieren des aufgenommenen Bildes, um die Form des Auges (121) des Individuums zu berechnen, und gegebenenfalls ferner Folgendes umfasst:
das Steuern der Maske (110) und der Lichtquelle (112), um das Lichtmuster über dem Auge (121) des Individuums abzutasten,
wobei die berechnete Form des Auges (121) des Individuums als Eingangsparameter zum Steuern des Abtastens verwendet wird, so dass ein Einfallswinkel des Maskenmusters des Auges (121) des Patienten während des gesamten Abtastvorgangs konstant gehalten wird; und/oder
das Aufnehmen als Bilderserie des Maskenmusters auf dem Auge (121) des Individuums durch die Bildgebungsvorrichtung (111) und
das Analysieren der Serie aufgenommener Bilder, um eine Tränenfilmstabilität und/oder Lipidschichtdicke in dem Auge (121) des Individuums zu bestimmen.

## Revendications

1. Appareil ophtalmologique (100) destiné à inspecter un œil, l'appareil comprenant :
une source de lumière (112) ;
un masque (110) conçu pour bloquer sélectivement une transmission de lumière à partir de la source de lumière (112) pour former un motif de masque qui peut être projeté sur un œil (121) d'un sujet, dans lequel le masque (110) peut être ajusté sur la base d'un signal de commande numérique de modification du motif de masque ; et
un dispositif d'imagerie (111) destiné à visualiser le motif de masque sur l'œil du sujet,
**caractérisé en ce que**
la source de lumière (112) comprend une partie émettrice de lumière à partir de laquelle de la lumière est projetée hors de l'appareil, et dans lequel une position relative de la partie émettrice de lumière et du masque (110) peut être commandée pour permettre l'ajustement d'un angle d'incidence du motif de masque pouvant être projeté sur l'œil (121) du sujet.

2. Appareil ophtalmologique (100) selon la revendication 1, dans lequel le masque (110) comprend un réseau d'éléments de masquage pouvant être commandés indépendamment, chaque élément de masquage étant ajustable, sur la base du signal de commande numérique, entre un état transmissif destiné à transmettre de la lumière à partir de la source de lumière (112) et un état opaque destiné à bloquer la transmission de lumière à partir de la source de lumière (112), et éventuellement :
dans lequel le réseau est monté dans un cadre (425) destiné à être placé dans un trajet optique de la source de lumière (112) à l'œil (121) du sujet, et/ou
dans lequel chaque élément de masquage pouvant être commandé indépendamment est adressable indépendamment.

3. Appareil ophtalmologique (100) selon la revendication 1 ou la revendication 2, dans lequel le masque comprend une fenêtre d'affichage à cristaux liquides.

4. Appareil ophtalmologique (100) selon la revendication 1, dans lequel la source de lumière (112) inclut un réseau d'éléments d'éclairage pouvant être activés sélectivement, et dans lequel la position relative de la partie émettrice de lumière et du masque (110) peut être commandée par activation sélective d'un ou de plusieurs des éléments d'éclairage ; ou
dans lequel la source de lumière (112) inclut un élément d'éclairage (116) et un masque secondaire (117) conçu pour bloquer sélectivement la transmission de lumière à partir de l'élément d'éclairage (116), et dans lequel la position relative de la partie émettrice de lumière et du masque (110) peut être commandée en ajustant une position d'une région de transmission de lumière du masque secondaire (117) par rapport à l'élément d'éclairage, et éventuellement dans lequel le masque secondaire (117) peut être commandé sur la base d'un second signal de commande numérique.

5. Appareil ophtalmologique (100) selon l'une quelconque des revendications précédentes, dans lequel la source de lumière (112) est fournie par des éléments d'éclairage dans l'affichage d'une tablette numérique ou d'un téléphone intelligent.

6. Appareil ophtalmologique (100) selon l'une quelconque des revendications précédentes, comprenant un polariseur conçu pour polariser de la lumière à partir de la source de lumière (112), et
éventuellement le polariseur est fourni dans le masque (110) et/ou la source de lumière (112).

7. Appareil ophtalmologique (100) selon l'une quelconque des revendications précédentes, dans lequel le dispositif d'imagerie (111) inclut :
un capteur d'image destiné à capturer une image numérique,
un alignement optique conçu pour focaliser une image du motif de masque qui est formée sur l'œil du sujet sur le capteur d'image, et
un affichage destiné à afficher l'image numérique capturée, et
éventuellement le capteur d'image comprend une paire de capteurs d'image indépendants disposés dans une relation espacée pour permettre à des images stéréoscopiques d'être détectées.

8. Appareil ophtalmologique (100) selon l'une quelconque des revendications précédentes, dans lequel le dispositif d'imagerie (111) inclut une caméra conçue pour enregistrer une image du motif de masque sur l'œil du sujet, et/ou
dans lequel le dispositif d'imagerie (111) est disposé à l'arrière du masque (110) par rapport à la direction dans laquelle le motif de masque est émis à travers lui, et dans lequel le masque inclut une ouverture pour permettre au dispositif d'imagerie de visualiser le motif de masque sur l'œil (121) du sujet.

9. Appareil ophtalmologique (100) selon l'une quelconque des revendications précédentes, dans lequel le masque (110) est contenu à l'intérieur d'un premier panneau et la source de lumière (112) est contenue à l'intérieur d'un second panneau, les deux panneaux étant agencés de telle sorte qu'ils sont sensiblement parallèles.

10. Appareil ophtalmologique (100) selon l'une quelconque des revendications précédentes, comprenant un dispositif de commande conçu pour générer le signal de commande numérique,
dans lequel, éventuellement, le dispositif de commande inclut un processeur et une mémoire stockant des instructions logicielles, dans lequel, lorsqu'elles sont exécutées par le processeur, les instructions logicielles sont conçues pour commander le fonctionnement de l'appareil et/ou le dispositif de commande est connecté opérationnellement au dispositif d'imagerie (111) pour commander la capture d'une image du motif de masque.

11. Unité modulaire pour utilisation en tant que partie d'un appareil ophtalmologique (100), l'unité modulaire comprenant :
un cadre (425) destiné à être placé de manière adjacente à un œil (121) d'un sujet, le cadre (425) comprenant :
un masque (110) monté sur celui-ci ;
une première partie de montage (427a, 427b) conçue pour recevoir une source de lumière (112), la source de lumière (112) comprenant une partie émettrice de lumière à partir de laquelle de la lumière est projetée, sur le côté opposé du masque (100) à partir de l'œil (121) du sujet ;
une seconde partie de montage (428) conçue pour recevoir un dispositif d'imagerie (111), dans lequel la seconde partie de montage (428) est conçue pour placer le dispositif d'imagerie (111) dans une position pour visualiser le motif de masque sur l'œil (121) du sujet,
dans lequel, lorsque la source de lumière est montée dans la première partie de montage,
le masque (110) est conçu pour bloquer sélectivement la transmission de lumière à partir de la source de lumière (112) pour former un motif de masque qui peut être projeté sur un œil (121) du sujet,
**caractérisé en ce que**
le masque (110) peut être ajusté sur la base d'un signal de commande numérique pour permettre un ajustement du motif de masque et dans lequel une position relative de la partie émettrice de lumière et du masque peut être commandée pour permettre un ajustement d'un angle d'incidence du motif de masque pouvant être projeté sur l'œil du sujet.

12. Unité modulaire selon la revendication 11, dans laquelle le masque (110) est fixé de manière amovible au cadre, et/ou
dans laquelle la seconde partie de montage (428) est conçue pour recevoir une tablette numérique ou un téléphone intelligent, dans laquelle la tablette numérique ou le téléphone intelligent fournit la source de lumière et/ou le dispositif d'imagerie.

13. Procédé de visualisation de la surface d'un œil (121) d'un sujet, le procédé incluant :
la mise en place d'un masque (110) entre l'œil (121) du sujet et une source de lumière (112) ;
la transmission d'un signal de commande numérique au masque (110) pour bloquer sélectivement la transmission de lumière à partir de la source de lumière et projeter ainsi un motif de masque sur l'œil (121) du sujet ; et
la visualisation, par un dispositif d'imagerie (111), du motif de masque sur l'œil (121) du sujet,
**caractérisé en ce que**
la source de lumière (112) comprend une partie émettrice de lumière à partir de laquelle de la lumière est projetée, et dans lequel le procédé inclut la commande d'une position relative de la partie émettrice de lumière et du masque (110) pour sélectionner ou ajuster un angle d'incidence du motif de masque projeté sur l'œil (121) du sujet.

14. Procédé selon la revendication 13 incluant :
le déplacement du motif de masque à travers l'œil (112) du sujet, et
la commande de l'angle d'incidence du motif de masque lorsqu'il est déplacé sur l'œil du sujet de sorte qu'il est sensiblement normal à une surface de l'œil ; et/ou
l'enregistrement, par le dispositif d'imagerie, d'une image du motif de masque sur l'œil (121) du sujet ;
l'analyse de l'image enregistrée ; et
sur la base de l'analyse de l'image enregistrée, la commande d'un ou de plusieurs du masque (110), de la source de lumière (112) et du dispositif d'imagerie (111) pour ajuster un paramètre de mesure.

15. Procédé selon la revendication 13 ou la revendication 14, incluant en outre :
la projection d'un motif de masque de dimensions connues sur l'œil (121) du sujet, l'œil (121) du sujet étant placé à une distance connue du dispositif d'imagerie (111) ;
l'enregistrement, à l'aide du dispositif d'imagerie (111), d'une image du motif de masque sur l'œil (121) du sujet ; et
l'analyse de l'image enregistrée, afin de calculer la forme de l'œil (121) du sujet, et incluant en outre éventuellement :
la commande du masque (110) et de la source de lumière (112) afin de balayer le motif de lumière à travers l'œil (121) du sujet,
dans lequel la forme calculée de l'œil (121) du sujet est utilisée comme un paramètre d'entrée pur commander le balayage, de telle sorte qu'un angle d'incidence du motif de masque sur l'œil (121) du patient est maintenu constant tout au long du balayage ; et/ou
l'enregistrement, par le dispositif d'imagerie (111), d'une série d'images du motif de masque sur l'œil (121) du sujet, et
l'analyse de la série d'images enregistrées afin de déterminer une stabilité du film lacrymal et/ou une épaisseur de couche lipidique dans l'œil (121) du sujet.
